# EUROPÄISCHE PATENTANMELDUNG

(11) **EP 3 012 320 A1**
(43) Veröffentlichungstag der Anmeldung: **27.04.2016**
(21) Anmeldenummer: 14189491.5
(22) Anmeldetag: 20.10.2014
(51) Int. Cl.: C12M 1/107, C12M 1/24, C12M 1/06, C12M 1/00, C12M 1/02

(54) **Fermenter**

(71) Anmelder: Innovative Biogas GmbH & Co. KG, 23909 Ratzeburg (DE)
(72) Erfinder: Liermann, Kai, 19294 Neu Kaliss (DE); Flaschka, Klaus, 23909 Ratzeburg (DE)
(74) Vertreter: Raffay & Fleck

(57) **Zusammenfassung**

Für einen Fermenter (1) für eine Anlage zur Umwandlung organischen Ausgangsmaterials in methanhaltiges Biogas mit:
- einem eine kreiszylinderförmige umlaufende Seitenwand (5) und einen diese nach unten hin abschließenden Boden (4) aufweisenden, ein Aufnahmevolumen (3) definierenden Fermenterbehältern (2);
- einer Abdeckung (6) zum gasdichten Abschluss des Fermenterbehälters (2) und zum Zurückhalten von entstehendem Biogas;
- einem Rührwerk zum Umwälzen von in dem Aufnahmevolumen (3) befindlichem organischen Ausgangsmaterial und in diesem befindlichen bioaktiven Kulturen;
- einer Speiseleitung zum Zuführen frischen organischen Ausgangsmaterials in das Aufnahmevolumen (3);
- einer Heizung zum Erwärmen und/oder Warmhalten des in dem Aufnahmevolumen (3) befindlichen organischen Ausgangsmaterials;
- einem Ablauf für verbrauchtes organisches Ausgangsmaterial;
- einem Abzug zur Entnahme von Biogas aus dem Fermenterbehälter (2) und
- einer Belüftung zum Zuführen von Luft in das Aufnahmevolumen (3) zum Belüften des darin gehaltenen organischen Ausgangsmaterials,

wird für eine weitere Optimierung von dessen Effizienz und Ausbeute an methanhaltigem Biogas vorgeschlagen,
dass das Aufnahmevolumen (3) durch den Boden (4) und die zylindrische Seitenwand (5) glattwandig und ohne jegliche Anbauteile an Boden (4) und/oder Seitenwand (5) in diesem Bereich begrenzt ist,
dass die Heizung eine Fußbodenheizung in dem Boden (4) umfasst,
dass das Rührwerk durch einen um eine Längsachse (14) der zylinderförmigen Seitenwand (5) rotierbaren Gitterrahmenrührer (15) gebildet ist mit Rührgitterrahmen, die sich ausgehend von der Längsachse (14) über im Wesentlichen die vollständige Füllhöhe des Aufnahmevolumens (3) und, zumindest im Bereich des Bodens (4), über im Wesentlichen den gesamten Radius des durch die Seitenwand (5) seitlich begrenzten Aufnahmevolu-mens (3) erstrecken,
dass die Speiseleitung zum Zuführen frischen organischen Ausgangsmaterials in das Aufnahmevolumen (3) wenigstens eine Druck für die Zufuhr des Ausgangsmaterials aufbauende Druckpumpe aufweist und dass die Speiseleitung in wenigstens einer, vorzugsweise mehreren oberhalb des Aufnahmevolumens (3) und oberhalb des Gitterrahmenrührers (15) angeordneten Einspritzdüsen mündet.

## Beschreibung

Die vorliegende Erfindung betrifft einen Fermenter für eine Anlage zur Umwandlung organischen Ausgangsmaterials in methanhaltiges Biogas.

Derartige Fermenter sind weitläufig bekannt und werden vielfach im Rahmen von sogenannten Biogasanlagen eingesetzt, um durch Umwandlung organischen Ausgangsmaterials methanhaltiges Biogas zu erhalten, welches in anschließenden Verfahrensschritten zur Gewinnung elektrischen Stroms sowie von Wärmeenergie genutzt wird.

Typischerweise werden in derartigen Fermentern organische Ausgangsmaterialien unterschiedlicher Herkunft und unterschiedlicher Art umgesetzt, bzw. werden solche Fermenter mit organischen Ausgangsmaterialien unterschiedlicher Art beschickt. Vielfach werden dabei organische Abfall- bzw. Restprodukte aus landwirtschaftlicher Produktion verwendet, so insbesondere Gülle oder Jauche. Es werden aber auch eigens für die Fermentierung und Biogaserzeugung bereitgestellte organische Ausgangsstoffe eingesetzt, wie z.B. Maishäcksel von eigens für die Biogasgewinnung angebautem Mais, aber auch Kartoffelschnitzel oder dgl. Auch sind Ansätze bekannt, hier mit anderen organischen Ausgangsmaterialien, z.B. Bioabfällen aus Haushalten, Gartenabfällen oder dgl. in derartigen Fermentern eine entsprechende Biogasgewinnung zu betreiben.

Typische bekannte Fermenter umfassen einen Fermenterbehälter, der ein Aufnahmevolumen umfasst, welches von einer kreiszylinderförmigen umlaufenden Seitenwand umgeben und nach unten hin durch einen Boden abgeschlossen ist. Weiterhin haben diese Fermenter eine Abdeckung, häufig eine flexible Abdeckung, die zweilagig und zumeist aus Folie gebildet ist, mit der der Fermenterbehälter gasdicht abgeschlossen ist und entstehendes Biogas zurückgehalten werden kann. Das Volumen unterhalb dieser Abdeckung, die ein gewisses Fassungsvermögen aufweist, ist zugleich auch eine Art Pufferspeicher für Biogas, bevor dieses abgesaugt und weiterverwertet wird.

Weiterer wesentlicher Bestandteil bestehender Fermenter ist ein Rührwerk, mit dem in dem Aufnahmevolumen befindliches organisches Ausgangsmaterial und die weiterhin in dem Aufnahmevolumen angeordneten, dem organischen Ausgangsmaterial zugesetzten bioaktiven Kulturen umgewälzt werden, um hier eine möglichst gute Durchmischung zu erhalten und ein Verklumpen von organischem Ausgangsmaterial und/oder Biofilm, der die bioaktiven Kulturen trägt, zu verhindern.

Weiterhin weisen die bekannten Fermenter jeweils wenigstens eine Speiseleitung zum Zuführen frischen organischen Ausgangsmaterials in das Aufnahmevolumen sowie einen Ablauf für verbrauchtes organisches Ausgangsmaterial aus dem Aufnahmevolumen auf.

Weiterhin verfügen die bekannten Fermenter über eine Heizung, mit der das in dem Aufnahmevolumen befindliche organische Ausgangsmaterial erwärmt bzw. auf einer vorgebbaren Temperatur gehalten werden kann. Auf diese Weise soll hinsichtlich der Temperatur ein optimales Milieu für die biologische Zersetzung bzw. Umsetzung des Ausgangsmaterials in Zersetzungsprodukte, hierbei insbesondere das methanhaltige Biogas, erhalten werden.

Weiterhin ist ein Abzug zur Entnahme von Biogas aus dem Fermenterbehälter den bekannten Fermentern eigen, und viele der bekannten Fermenter verfügen über eine Belüftung zum Zuführen von Luft in das Aufnahmevolumen für ein Belüften des darin gehaltenen organischen Ausgangsmaterials. Mit einer solchen Belüftung soll der für die Umsetzung des organischen Ausgangsmaterials erforderliche Sauerstoff in das Aufnahmevolumen des Fermenterbehälters eingebracht werden.

Bekannte Fermenter der eingangs aufgezeigten Art werden, wie bereits erwähnt, bereits vielfach im Zusammenhang mit sogenannten Biogasanlagen zur Gewinnung erneuerbarer Energien eingesetzt. Typische Auslegungen derartiger Biogasanlagen werden für die Versorgung kleinerer bis mittelgroßer Einheiten gewählt, d.h. beginnend mit der Versorgung einer landwirtschaftlichen Hofanlage bis hin zu der Versorgung ganzer ländlicher Gemeinden. Entsprechend liegen die Kapazitäten bzw. Leistungen derartiger Biogasanlagen häufig zwischen 500 Kilowatt bis hinein in den Megawattbereich. Entsprechende Fermenter haben dabei beispielsweise Abmessungen des Aufnahmevolumens mit Durchmessern von ca. 20 m und Höhen von bis zu 10 m. Es gibt aber im Stand der Technik auch bereits Ansätze und Überlegungen, entsprechende Biogasanlagen mit geringerer Leistung zu skalieren, wobei hier dann entsprechend kleiner dimensionierte Fermenter zum Einsatz kommen sollen. Ein Beispiel hierfür ist in der DE 20 2007 017 166 U1 angegeben.

Die Erfinder haben nun erkannt, dass für die aus dem Stand der Technik bekannten Fermenter und deren Einsatz in Biogasanlagen weiterhin erheblicher Optimierungsbedarf besteht, um eine deutlich verbesserte Ausbeute an erhaltenem methanhaltigen Biogas pro eingesetzter Einheit an organischem Ausgangsmaterial zu erzielen. Mit dieser Optimierungsaufgabe haben sich die Erfinder der vorliegenden Erfindung befasst und sind dabei zu dem hier beanspruchten und nachstehend beschriebenen Erfindungsgegenstand, einem neuartigen Fermenter, gelangt.

Dieser neuartige und erfindungsgemäße Fermenter für eine Anlage zur Umwandlung organischen Ausgangsmaterials in methanhaltiges Biogas hat in Übereinstimmung mit dem Stand der Technik einen eine kreiszylinderförmige umlaufende Seitenwand und einen diese nach unten hin abschließenden Boden aufweisenden, ein Aufnahmevolumen definierenden Fermenterbehälter; eine Abdeckung zum gasdichten Abschluss des Fermenterbehälters und zum Zurückhalten von entstehendem Biogas; ein Rührwerk zum Umwälzen von in dem Aufnahmevolumen befindlichem organischen Ausgangsmaterial und in diesem befindlichen bioaktiven Kulturen; eine Speiseleitung zum Zuführen frischen organischen Ausgangsmaterials in das Aufnahmevolumen; eine Heizung zum Erwärmen und/oder Warmhalten des in dem Aufnahmevolumen befindlichen organischen Ausgangsmaterials; einen Ablauf für verbrauchtes organisches Ausgangsmaterials; einen Abzug zur Entnahme von Biogas aus dem Fermenterbehälter und eine Belüftung zum Zuführen von Luft in das Aufnahmevolumen zum Belüften des darin gehaltenen organischen Ausgangsmaterials.

In erfindungsgemäßer Weise ist bei dem weiterentwickelten und optimierten Fermenter das Aufnahmevolumen durch den Boden und durch die zylindrische Seitenwand glattwandig und ohne jegliche Anbauteile am Boden und/oder an der Seitenwand in diesen Bereich begrenzt. Dies bedeutet, dass keine vorstehenden Elemente über den Verlauf der Seitenwand bzw. des Bodens in Richtung des Aufnahmevolumens vorstehen. Durch diese Maßnahme wird erreicht, dass ansonsten häufig anzutreffende Quellen für Verwirbelungen der in dem Aufnahmevolumen enthaltenen Masse aus organischem Ausgangsmaterial, bioaktiven Kulturen sowie ggf. einem Lösungsmittel (z.B. Wasser) entstehen und somit vermittelt durch das Rührwerk eine besonders gleichmäßige und verwirbelungsfreie und somit für die Ausbeute effiziente Durchmischung des organischen Ausgangsmaterials ermöglicht ist.

Weiterhin ist für den erfindungsgemäßen Fermenter vorgesehen, dass die Heizung eine Fußbodenheizung in dem Boden umfasst. Eine solche flächige Heizung führt nicht nur dazu, dass eine Erwärmung und ein Halten des organischen Ausgangsmaterials in dem Aufnahmevolumen auf einer vorgegebenen Temperatur ohne in das Innere des Fermenterbehälters vorstehende Elemente erfolgen kann, sondern auch, dass diese Erwärmung besonders gleichmäßig und effizient erfolgt durch eine große für den Wärmeaustausch vorgesehene Fläche, nämlich den im Wesentlichen gesamten Boden des Fermenters.

Weiterhin ist bei dem erfindungsgemäßen Fermenter vorgesehen, dass das Rührwerk durch einen um eine Längsachse der zylinderförmigen Seitenwand rotierbaren Gitterrahmenrührer gebildet ist mit Rührgitterrahmen, die sich ausgehend von der Längsachse über im Wesentlichen die vollständige Füllhöhe des Aufnahmevolumens und, zumindest im Bereich des Bodens, über im Wesentlichen den gesamten Radius des durch die Seitenwand seitlich begrenzten Aufnahmevolumens erstrecken. Ein derartiger Gitterrahmenrührer stellt ein von den bis dahin bekannten und eingesetzten Rührerkonzepten abweichendes Rührerkonzept dar. Im Stand der Technik wurden bisher vielfach propeller- oder schaufelradartige Rührer eingesetzt, die einen großen lokalen Impuls auf die in dem Fermenter bzw. in dem Aufnahmevolumen befindliche Masse aus organischem Ausgangsmaterial aufbringen (vgl. hierzu z.B. DE 20 2006 020 742 U1) und hierfür mit großer Umdrehungszahl in Rotation versetzt werden. Diese Art der Rührer bedingen aber eine stark unterschiedliche und zur Ausbildung von Verwirbelungen neigende Strömung, die in dem Fermenterbehälter in seinem Aufnahmevolumen induziert wird, was - wie die Erfinder herausgefunden haben - sich nachteilig für die Umsetzung des organischen Ausgangsmaterials zu methanhaltigem Biogas auswirkt. Denn die bioaktiven Kulturen können, dies haben Experimente der Erfinder aufgezeigt, deutlich besser und mit deutlich verbesserter Effizienz organisches Ausgangsmaterials unter Bildung von methanhaltigem Biogas umsetzen, wenn zwar eine stetige Umwälzung des Inhaltes des Aufnahmevolumens erfolgt, dies jedoch ohne hohe Strömungsgeschwindigkeiten und ohne Verwirbelungen.

Weiterhin sieht die Erfindung vor, dass bei dem Fermenter die Speiseleitung zum Zuführen frischen organischen Ausgangsmaterials in das Aufnahmevolumen wenigstens eine Druck für die Zufuhr des Ausgangsmaterials aufbauende Druckpumpe aufweist und die Speisleitung in wenigstens einer, vorzugsweise mehreren oberhalb des Aufnahmevolumens und oberhalb des Gitterrahmenrührers angeordneten Einspritzdüsen mündet. Über diese Maßnahme wird frisches in das Aufnahmevolumen des Fermenterbehälters einzubringendes organisches Material mit Druck auf die Oberfläche des bereits in dem Fermenterbehälter befindlichen organischen Materials aufgesprüht, bzw. über die Oberfläche in das Volumen des Fermenterbehälters eingedrückt. Diese Art der Speisung und Zufuhr hat sich als besonders effektiv erwiesen, da sie zum einen bereits mit der Zufuhr des organischen Ausgangsmaterials eine gute Durchmengung des "frischen" Materials mit dem bereits im Fermenter befindlichen Material bewirkt, andererseits jedoch keine zusätzlichen und störenden Verwirbelungen nach sich zieht, wie sie durch eine unmittelbare in das Aufnahmevolumen mündende Einspeiseleitung ansonsten erhalten werden.

Durch die oben aufgezeigten erfindungsgemäßen Optimierungen des Fermenters konnten so erhebliche Effizienzsteigerungen erzielt werden, die in einem auf Versuchsanlagengröße skalierten Vergleich bei Steigerungen um bis zu über 85% lagen. Einen wesentlichen Anteil dieser Effizienzsteigerung schreiben die Erfinder dabei dem erfindungsgemäßen Rührer zu, der insbesondere mit geringen Drehzahlen im kontinuierlichen Betrieb für eine durchgehende und weitgehend verwirbelungsfreie Durchmischung des in dem Aufnahmevolumen befindlichen Ausgangsmaterials sorgt. Hierbei wurden Drehzahlen des Rührers im Bereich zwischen 1 und 5 Umdrehungen pro Minute eingestellt, im Besonderen zwischen 2 und 3 Umdrehungen pro Minute, mit besonderem Vorteil und günstigen Ergebnissen von etwa 2,5 Umdrehungen pro Minute. Durch die spezielle Ausgestaltung des Rührers als Gitterrahmenrührer mit über die Höhe wie auch über den Radius verteilt angeordneten durchmengungswirksamen Elementen wurde eine Durchmischung über das gesamte Aufnahmevolumen erreicht, so dass einerseits eine gute Durchmengung des organischen Ausgangsmaterials mit den bioaktiven Kulturen erreicht wurde, andererseits möglichen Verklumpungen im gesamten Volumen vorgebeugt und somit eine große oberflächenmäßige Durchdringung der biologischen Ausgangsmaterialien mit den bioaktiven Kulturen erzielt wurde. Diese Maßnahme zusammen mit dem Umstand, dass durch die glattwandige Bauweise weiterhin potentielle Auslöser für Verwirbelungen ausgemerzt wurden, führten so zu einer sehr effizienten Umsetzung des organischen Ausgangsmaterials mit den bioaktiven Kulturen zu methanhaltigem Biogas.

Die besondere Steigerung der Effizienz erklären sich die Erfinder damit, dass hier durch das permanente Umwälzen einerseits eine gute Durchmengung und Durchmischung des Ausgangsmaterials mit den bioaktiven Kulturen gegeben ist, so dass diese Kulturen effektiv eine Zersetzung des organischen Materials unter Bildung von Biogas betreiben können, dass andererseits nicht zu schnelle Strömungen und Verwirbelungen, wie sie bei Fermentern nach dem Stand der Technik erzielt werden, zu einem "Stressfaktor" für die biologisch aktiven Substanzen führen, die dann zu einer Verringerung der Effizienz führen. Es werden mit anderen Worten für die biologisch aktiven Kulturen die optimalen Voraussetzungen für eine effiziente Umsetzung des in dem Fermenter in dem Aufnahmevolumen enthaltenen organischen Ausgangsmaterials erreicht und geboten.

Gemäß einer vorteilhaften Weiterbildung der Erfindung können die das Aufnahmevolumen begrenzenden Oberflächen der Seitenwand und des Bodens für eine Haftungsverminderung behandelt, insbesondere beschichtet, sein. Eine entsprechende haftungsvermindernde Behandlung, so z.B. eine Beschichtung oder eine Ausprägung mit den sogenannten Lotoseffekt ausbildenden Strukturen, verhindert hier das Anwachsen von Biofilmakkumulationen, die wiederum Quellen und Auslöser für Verwirbelungen sein können. Darüber hinaus bleiben die bioaktiven Kulturen in Suspension und sind dort für die Umsetzung des organischen Ausgangsmaterials zu methanhaltigem Biogas aktiv.

Gemäß einer weiteren vorteilhaften Weiterbildung kann bei dem Fermenter nach der Erfindung die Belüftung eine Luftführung im Boden oder eine Vielzahl von in dem Boden gebildeten Eingangsdüsen umfassen, wobei in dem Boden geführte Luftleitungen so in Kontakt mit in dem Boden eingelassenen Rohren der Fußbodenheizung gebracht sind, dass im Betrieb durch die in den Rohren der Fußbodenheizung geführtes Heizmedium die Luft in den Luftleitungen vorerwärmt wird, bevor diese durch die Eingasdüsen das Aufnahmevolumen einströmt. Durch das Eingasen der Belüftung durch eine Vielzahl von Eingasdüsen in das Aufnahmevolumen wird eine feinperlige und hinsichtlich der Blasenoberfläche großflächige Einleitung von Luft, insbesondere Sauerstoff, in das biologische Ausgangsmaterial in dem Aufnahmevolumen erreicht, so dass es hier schnell zu einer aktivierenden und die Umsetzung des organischen Ausgangsmaterials unterstützenden Wirkung kommt. Zudem wird durch das Vorerwärmen der Luft verhindert, dass durch den Eintrag derselben eine, ggf. auch nur lokale, Abkühlung des in dem Aufnahmevolumen befindlichen biologischen Ausgangsmaterials die Folge ist. Denn eine solche Abkühlung führt, dies haben die Erfinder herausgebracht, unvermittelt zu einer Verschlechterung der Effizienz, da die in dem Fermenter vorhandenen biologisch aktiven Kulturen ihre höchste Umsetzungseffizienz in einem gegebenen Temperaturfenster erreichen, diese Effizienz sogleich abnimmt, wenn die Temperatur den unteren Schwellwert dieses Fensters unterschreitet. Für die wie geschilderte Vorerwärmung der Luft können beispielsweise die Luftzuleitungen konzentrisch um die in den Boden eingelassenen Rohre der Fußbodenheizung herum gelegt sein, so dass die von dem in den Rohren geführten Heizmedium abgegebene Wärme zunächst die zugeführte Luft erwärmt, sodann auch über den Boden das in dem Aufnahmevolumen befindliche organische Ausgangsmaterial.

Gemäß einer weiteren vorteilhaften Weiterbildung der Erfindung kann der Gitterrahmenrührer zweiflügelig und symmetrisch zu der Längsachse gebildet sein. Ein solcher zweiflügeliger Gitterrahmenrührer, der mithin auch durch einen einzigen, sich über den gesamten Durchmesser des Fermenters erstreckenden Gitterrahmenflügel bzw. ein solches Gitterrahmenelement gebildet sein kann, hat sich als für eine für die Umsetzung optimale Durchmengung besonders geeignet erwiesen und ist mechanisch einfach konstruiert und stabil.

Weiterhin kann bei dem erfindungsgemäßen Fermenter gemäß einer weiteren vorteilhaften Weiterbildung vorgesehen sein, dass die Rührgitterrahmen des Gitterrahmenrührers je eine äußere Rahmenkonstruktion und dazwischen verlaufende Längs- und Querstreben aufweisen, wobei die Längsstreben im Wesentlichen vertikal, die Querstreben im Wesentlichen horizontal verlaufen. Mit dieser Art der Gestaltung des Gitterrahmenrührers wird, dies haben Versuche der Erfinder gezeigt, eine besonders effiziente und gute Durchmischung ohne Verwirbelungen in dem in dem Aufnahmevolumen befindlichen organischen Ausgangsmaterial erzielt. Dies gilt insbesondere dann, wenn, wie gemäß einer weiteren vorteilhaften Weiterbildung der Erfindung vorgesehen, in den jeweiligen Rührgitterrahmen die Längsstreben derart angeordnet sind, dass sie diesen in regelmäßigen Abständen entlang eines horizontalen Verlaufes von der Längsachse gesehen nach außen unterteilen. Weiterhin gilt dies im Besonderen, wenn wie gemäß einer weiteren vorteilhaften Weiterbildung der Erfindung vorgeschlagen die Längs- und Querstreben durch Flachbandmaterial gebildet sind und entlang ihrer jeweiligen Längsachse wenigstens einmal um 180° verdreht sind. Durch diese Verdrehung ergeben sich zum einen unterschiedliche Wirkflächen das bandförmigen Materials, zum anderen ergeben sich auch Leitstrukturen, mit denen das in dem Aufnahmevolumen befindliche organische Ausgangsmaterial aktiv umgewälzt wird, ohne dass dies etwa zu den als schädlich erkannten übermäßigen Verwirbelungen führen würde.

Weiterhin kann eine weitere vorteilhafte Ausgestaltung des erfindungsgemäßen Fermenters zu einer Optimierung desselben beitragen, gemäß derer ein Antriebsmotor für den Gitterrahmenrührer außerhalb des Fermenterbehälters angeordnet und über durch den Fermenterbehälter geführten Antriebswellen mit dem Gitterrahmenrührer wirkverbunden ist. Diese Gestaltung, bei der die Antriebswellen insbesondere außerhalb des Aufnahmevolumens geführt sind, trägt zu einer einfachen und ohne in das Innere des Aufnahmevolumens vorstehende Teile gebildeten Gestaltung des Fermenterbehälters bei, was wiederum hilft, Verwirbelungen beim Umwälzen des in dem Aufnahmevolumen enthalten organischen Ausgangsmaterials zu verhindern.

Neben der zwingend vorgesehenen Fußbodenheizung am Boden des Fermenterbehälters kann, wie dies eine weitere vorteilhafte Ausgestaltung des erfindungsgemäßen Fermenters vorsieht, bei diesem auch in der Seitenwand eine Wandheizung angeordnet sein zum Erwärmen bzw. zum Warmhalten, d.h. also zum geregelten Temperieren, des in dem Aufnahmevolumen befindlichen organischen Ausgangsmaterials. Diese Wandheizung kann dabei gemäß einer weiteren vorteilhaften Weiterbildung der Erfindung mit der Fußbodenheizung zu einem gemeinsamen Heizkreislauf verbunden sein.

Prinzipiell kann ein erfindungsgemäßer Fermenter in allen erdenklichen Größen skaliert und somit für Ausgangsleistungen unterschiedlicher Auslegung gebildet und verwendet werden. Es hat sich im Zusammenhang mit den Untersuchungen der Erfinder aber als besonders vorteilhaft erwiesen, dass der erfindungsgemäße Fermenter auch für kleintechnische Anlagen, d.h. solche mit geringer Leistung, die z.B. für die Versorgung eines typischen Einfamilienhauses ausgelegt sind (Leistungen im Bereich von 0,5 kW bis einige wenige kW) geeignet ist. Entsprechend kann ein erfindungsgemäßer Fermenterbehälter einen das Aufnahmevolumen begrenzenden Innendurchmesser von zwischen 2 und 2,5 m, insbesondere von 2,2 m und eine Füllhöhe für das Aufnahmevolumen von zwischen 0,6 m und 1,0 m, insbesondere von 0,8 m, aufweisen.

Die Abdeckung kann, entsprechend den aus dem Stand der Technik bekannten Abdeckungen, als flexible Abdeckung gebildet sein, insbesondere aus Folie, vorzugsweise zweilagig. Sie kann aber auch als starre Abdeckung, z.B. als plattenförmiger und flacher Abschluss, gebildet sein.

In einem weiteren Aspekt betrifft die Erfindung dann auch eine Biogasanlage, die wenigstens einen Fermenter nach einem der vorstehend beschriebenen Aspekte aufweist. Eine solche Biogasanlage zieht entsprechenden Nutzen aus den oben im Zusammenhang mit der Darlegung und Erläuterung des neuartigen Fermenters beschriebenen Vorteilen.

Der erfindungsgemäße Fermenter wird insbesondere mit einer eigens aufbereiteten Suspension eines organischen Ausgangsmaterials betrieben, wobei diese Suspension das organische Ausgangsmaterial in besonders feinteiliger Größe bzw. Körnung enthält. Besonders gute Ergebnisse konnten die Erfinder erzielen mit einem auf Basis von Kartoffeln hergestellten brei- bzw. feinteilig suspensionsförmigen organischen Ausgangsmaterial. Die Temperatur dieses organischen Ausgangsmaterials im Aufnahmevolumen des Fermenters wurde bei besonders erfolgreichen Versuchen in einer Größenordnung zwischen 37°C und 39°C, insbesondere zwischen 37,5°C und 38°C eingestellt. Weiterhin haben die Erfinder für eine Entschwefelung des Biogases bereits im Fermenter eine Zink-Opfer-Elektrode angeordnet, die dafür sorgte, dass das über den Abzug entnommene Biogas besonders schwefelarm war.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: eine perspektivische, teilweise weggeschnittene Ansicht eines erfindungsgemäßen Fermenters in einer ersten Gestaltungsvariante;
- Fig. 2: eine Detailansicht, die die Anbindung der flexiblen Abdeckung des Fermenters an die umlaufende Seitenwand bei dem in Fig. 1 gezeigten Ausführungsbeispiel eines erfindungsgemäßen Fermenters darstellt;
- Fig. 3: eine Detaildarstellung der Anbindung der Seitenwand an dem Boden bei dem in Fig. 1 dargestellten Ausführungsbeispiel eines erfindungsgemäßen Fermenters;
- Fig. 4: eine perspektivische, teilweise weggeschnittene Ansicht eines erfindungsgemäßen Fermenters in einer zweiten Gestaltungsvariante;
- Fig. 5: eine Detailansicht, die die Anbindung der starren, plattenförmigen Abdeckung des Fermenters an die umlaufende Seitenwand bei dem in Fig. 4 gezeigten Ausführungsbeispiel eines erfindungsgemäßen Fermenters darstellt; und
- Fig. 6: eine Ansicht des Gitterrahmenrührers in einer möglichen Ausgestaltung, wie er in dem in Fig. 1 dargestellten Ausführungsbeispiel eines erfindungsgemäßen Fermenters Verwendung findet.

In den Figuren sind mögliche Ausgestaltungsvarianten der Erfindung in schematisch veranschaulichender Weise dargestellt, wobei die Figuren keineswegs maßstabsgerechte Darstellungen oder vollständige Konstruktionszeichnungen sind. Anhand dieser Figuren werden nun nachstehend Ausführungsbeispiele der Erfindung beschrieben, wobei die nachstehend beschriebenen einzelnen und gesonderten Merkmale sich nicht allein auf die geschilderten Ausführungsbeispiele beschränken, sondern, sofern nichts anderes angegeben, auch allgemein und in anderer Kombination für die Verwirklichung der Erfindung relevant sind bzw. sein können.

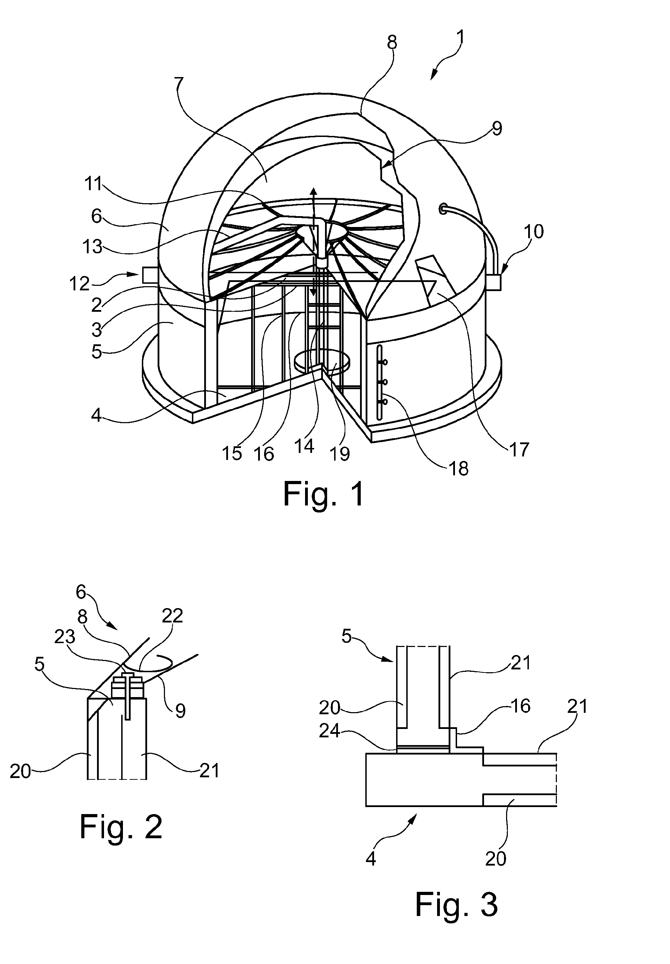

In Fig. 1 ist zunächst ein erfindungsgemäß gestalteter Fermenter in einer ersten Gestaltungsvariante gezeigt und allgemein mit der Bezugsziffer 1 bezeichnet. Der Fermenter 1 weist einen Fermenterbehälter 2 auf, der ein Aufnahmevolumen 3 umschließt. Dieser Fermenterbehälter wird im Wesentlichen begrenzt durch eine Bodenplatte 4 und eine an dieser dichtend sich anschließende, zylinderförmig umlaufende Seitenwand 5.

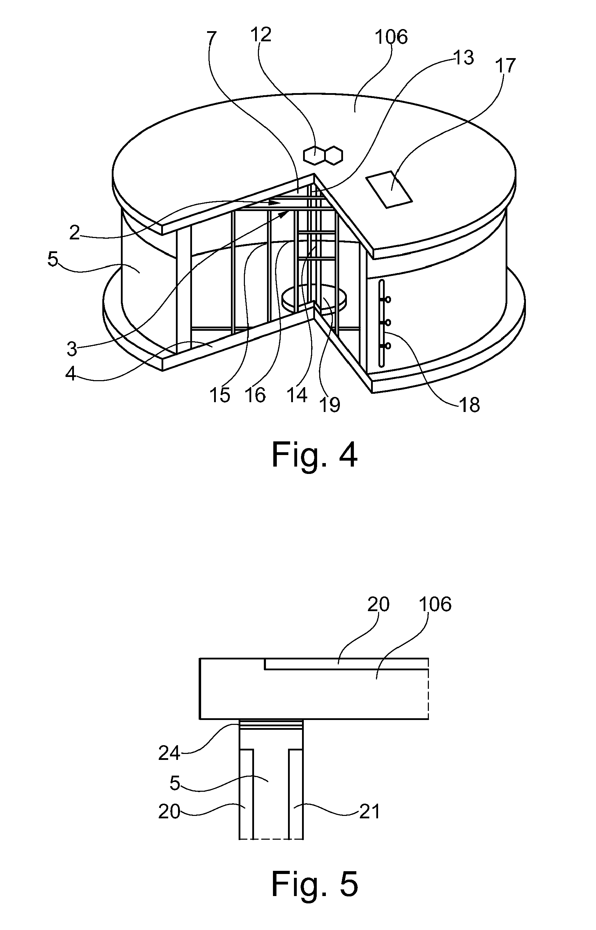

Auf die Seitenwand 5 aufgesetzt und mit dieser dichtend verbunden ist eine flexible Abdeckung 6, die das Aufnahmevolumen 3 und ein sich oberhalb desselben anschließendes Gasreservoir 7 gasdicht verschließt. Die flexible Abdeckung 6 setzt sich dabei zusammen aus einer äußeren Folie oder Membran 8 und einer inneren Folie oder Membran 9. In dem Bereich zwischen diesen beiden Membranen, äußere Membran 8 und innere Membran 9, wird über ein Gebläse 10 ein Überdruck eingeblasen, um so die flexible Abdeckung 6 in ihrer wie in der Fig. 1 gezeigten haubenförmigen Gestalt zu halten und zudem eine thermische Isolierung zu bilden und so einen übermäßigen Wärmeverlust aus dem Innern des Fermenterbehälters 2 zu verhindern. Im Inneren der flexiblen Abdeckung 6 ist oberhalb des Aufnahmevolumens 3 eine Netzabdeckung 11, beispielsweise ein Nylonnetz, aufgespannt, die ein Hereinfallen von Gegenständen in das Aufnahmevolumen 3 verhindern und auch eine Mannsicherung bilden soll. Von einem außerhalb der Seitenwand 5 angeordneten Antriebsmotor 12 und an diesen Antriebsmotor 12 angeschlossene Antriebswellen 13 zur Rotation um eine vertikale Mittelachse 14 des Aufnahmevolumens 3 angetrieben ist ein Gitterrahmenrührer 15. Dieser ist als durchgehendes Rahmenelement gebildet, welches auch als zwei einander gesetzte, in einem Winkel von 180° zueinander ausgerichtete Rührrahmengitter aufgefasst werden kann. Der Gitterrahmenrührer 15 erstreckt sich über die gesamte Höhe des Aufnahmevolumens 3 und über im Wesentlichen den gesamten Durchmesser desselben im Bereich der Bodenplatte 4. Er läuft auf einer am Rand der Bodenplatte 4 umlaufend verlaufend ausgebildeten Laufschiene 16 um und wird im Betrieb mit einer Drehzahl von etwa 2 bis 3, vorzugsweise 2,5 Umdrehungen pro Minute, kontinuierlich um die Mittelachse 14 rotierend bewegt. Der Gitterrahmenrührer 15 hat dabei einen trapezförmigen Rahmenaufbau mit zu seinem oberen Ende hin kürzerer Horizontalrahmenstreben, wie sich im Weiteren auch noch aus der genaueren Beschreibung der Fig. 4 ergeben wird.

Zu erkennen ist weiterhin noch mit 17 bezeichnet eine Revisionsöffnung, die hier geöffnet dargestellt, im Normalfall aber verschlossen ist und über die z.B. Überprüfungen oder einfache Wartungen durchgeführt werden können, ohne dass die gesamte flexible Abdeckung 6 zu entfernen ist. Weiterhin ist eine Füllstandsanzeige 18 dargestellt, über die von außerhalb des Fermenters 1 der Füllstand im Aufnahmevolumen 3 erkannt werden kann, um so ggf. regulierend eingreifen zu können entweder durch Absaugen überschüssigen organischen Ausgangsmaterials oder aber durch Nachführen frischen solchen organischen Ausgangsmaterials zum Auffüllen des Aufnahmevolumens 3.

Nicht näher dargestellt ist eine hier im mit 19 bezeichneten Bereich angeordnete Absaugung, über die bodennah dort sich ansammelndes verbrauchtes organisches Ausgangsmaterial und überschüssige biologisch aktive Kulturen aus dem Aufnahmevolumen 3 des Fermenters 1 abgezogen werden können. Ebenfalls nicht gezeigt sind die Speise- bzw. Zuführleitungen für frisches organisches Ausgangsmaterial, welche in oberhalb des Aufnahmevolumens 3 im Bereich unterhalb der Netzabdeckung 11 mündenden Einströmdüsen enden und so eine über die Fläche der Oberfläche des Aufnahmevolumens 3 verteilte mit Druck eingebrachte Einspeisung von organischem Ausgangsmaterial erlauben. Ebenfalls nicht näher eingezeichnet ist in der Fig. 1 die in der Bodenplatte 4 angeordnete Bodenheizung, über die das in dem Aufnahmevolumen 3 befindliche organische Ausgansmaterial erwärmt und auf eine Betriebstemperatur (typischerweise zwischen 37°C und 39°C, im Idealfall zwischen 37,5°C und 38°C) gehalten wird. Ebenfalls in dieser Darstellung nicht zu erkennen sind feine Eingasdüsen in der Bodenplatte 4, über die Luft in das Aufnahmevolumen 3 und das darin befindliche organische Ausgangsmaterial eingeblasen werden kann.

Ein weiterer wesentlicher Aspekt der Erfindung ist hier jedoch schon zu erkennen, nämlich der Umstand, dass die Begrenzung durch die das Aufnahmevolumen 3 umschließende Seitenwand 5 und Bodenplatte 4 glattwandig und ohne vorstehende Elemente gebildet ist, so dass hier keine etwa Verwirbelungen erzeugende Elemente in das Aufnahmevolumen 2 hineinragen.

In Fig. 2 ist in einer Ausschnittsdarstellung schematisch dargestellt, wie die flexible Abdeckung 6 mit ihrer äußeren Membran 8 und der inneren Membran 9 an der umlaufenden Seitenwand 5 festgelegt ist. Beide Membranen sind mit mittels Schraubbolzen 23 aufeinander zu gezogenen Klemmleisten gegenüber der Seitenwand 5 verklemmt und so fixiert. Weiterhin zu erkennen ist, dass die Seitenwand 5 auf ihrer Außenseite eine Isolierschicht 20 aufweist, die eine thermische Isolierung des Fermenterbehälters und insbesondere des Aufnahmevolumens 3 bewirkt. Auf der dem Aufnahmevolumen 3 zugewandten Innenseite ist auf der Seitenwand 5 eine Schutzbeschichtung 21 mit Antihaft-Effekt aufgebracht, bei der es sich z.B. um eine Schutzbeschichtung mit Lotoseffekt handeln kann und die ein Festsetzen bzw. Anwachsen von Biofilmen auf der Seitenwand 5 verhindert. Auch zwischen den beiden Membranen äußere Membran 8 und innere Membran 9 ist eine Isolierung 22 angeordnet, um auch in diesem Bereich Wärmeverluste möglichst zu vermeiden und somit einen energieeffizienten Betrieb des Fermenters zu gewährleisten.

In Fig. 3 ist in einer vergrößerten schematischen Darstellung die Anbindung der Seitenwand 5 an die Bodenplatte 4 gezeigt. Die Seitenwand 5 ist unter Zwischenlage einer Dichtung 24 auf die Bodenplatte 4 aufgesetzt und mit dieser verbunden. Zu erkennen sind hier wiederum Isolierschichten 20, die nicht nur auf der Seitenwand 5, sondern auch auf der dem Aufnahmevolumen 3 abgewandten Seite der Bodenplatte 4 angeordnet sind und die einen Wärmeverlust vermeiden sollen. Weiterhin ist gezeigt, dass nicht nur die Seitenwand 5, sondern auch die dem Innenvolumen 3 zugewandte Seite der Bodenplatte 4 mit einer Schutzbeschichtung 21 mit Antihaft-Effekt versehen ist, beispielsweise einer Beschichtung mit Lotoseffekt. Schließlich ist die Laufschiene 16 für den Gitterrahmenrührer 15 zu erkennen, die als Winkelprofil in den Stoßwinkel zwischen Seitenwand 5 und Bodenplatte 4 eingesetzt ist und den Verlauf der Seitenwand 5 kreisförmig folgt.

In Fig. 4 ist in einer der Fig. 1 vergleichbaren Darstellung eine abgewandelte Ausführungsform eines Fermenters 100 gezeigt. Dieser ist in den wesentlichen Elementen gleich aufgebaut, wie der Fermenter 1, wobei diese wesentlichen Elemente mit gleichen Bezugszeichen wie in Fig. 1 versehen sind und gleichartig aufgebaut und funktional zueinander eingerichtet sind. Insoweit kann auf die Beschreibung der Fig. 1 oben verwiesen werden, die - sofern nicht nachstehend eine abweichende Erläuterung gegeben ist - auch für den Fermenter 100 zutrifft. Der einzige relevante Unterschied besteht dabei in der Art der Abdeckung. Anders als bei dem Fermenter 1 ist bei dem Fermenter 100 die Abdeckung, die den Fermenterbehälter nach oben hin gasdicht abschließt, nicht eine flexible. Vielmehr ist die Abdeckung 106 hier als starre Platte, z.B. aus glasfaserverstärktem Kunststoff (GFK), carbonfaserverstärktem Kunststoff (CFK) oder aber auch Beton, gebildet. Bei dieser Ausgestaltungsvariante ist der Antriebsmotor 12 für den Gitterrahmenrüher 15 als auf der Abdeckung 106 angeordnet gezeigt. Dies erlaubt eine einfachere Führung der Antriebswelle 13, die als einfache Welle vertikal nach unten geführt werden kann und auf der dann der Gitterrahmenrührer 15 angeordnet ist. Zwischen dem Antriebsmotor 12 und der Antriebswelle 13 kann, wie auch in dem Ausführungsbeispiel nach Fig. 1, ein Getriebe angeordnet sein. Auch kann - und wird dies in der Regel auch - der Antriebsmotor 12 in beiden Ausgestaltungsvarianten mit einer Steuerung verbunden sein, die die Drehzahl regelt und ggf. Rühr- und Pausenzeiten vorgibt, wobei in den letztgenannten das Substrat (die organischen Ausgangsmaterialien in dem Aufnahmevolumen 3) ruhen kann.

In Fig. 5 ist in einer vergrößerten Darstellung die Anbindung zwischen umlaufender Seitenwand 5 und der Abdeckung 106 bei dem Fermenter 100 gezeigt. Zu erkennen ist, dass diese ähnlich aufgebaut ist, wie die Anbindung der Seitenwand 5 an den Boden 4 bei dem Fermenter 1 (vergleiche Fig. 3), welche letztgenannten Anbindung im Übrigen auch bei dem Fermenter 100 so aufgebaut ist, wie in Fig. 3 dargestellt. So ist zwischen der oben Stirnfläche der Seitenwand 5 und der Abdeckung 106 eine Dichtung 24 angeordnet. Auch ist in dieser Darstellung der Fig. 5 zu erkennen, dass die Abdeckung 106 auf ihrer oberen Fläche eine Isolierschicht 20 aufweist, die auch hier der Verhinderung von Wärmeverlusten aus dem Innern des Fermenterbehälters 100 dient. Die Abdeckung 106 kann z.B. bei solchen Fermentern 100 mit vergleichsweise geringen Maßen als ganze abnehmbar und z.B. mit Klemm- oder Schraubverschlüssen an der Seitenwand 5 befestigt sein. Dies erleichtert die Schaffung eines Zuganges zum Aufnahmevolumen 3, um dort ggf. Wartungs- oder Reinigungsarbeiten durchführen zu können.

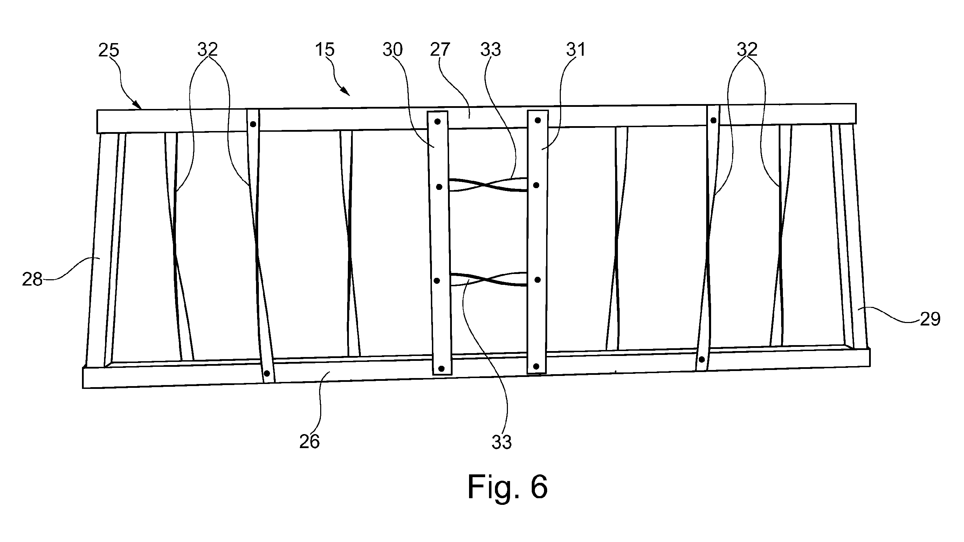

In Fig. 6 ist schließlich noch einmal der Gitterrahmenrührer 15 losgelöst von dem Fermenter dargestellt. Zu erkennen ist, dass dieser zunächst einen trapezförmigen Rahmen 25 aufweist, der aus U-Profilen aus einem Metall, insbesondere einem Edelstahl, aufgebaut ist. Der trapezförmige Rahmen weist zwei parallel zueinander verlaufende horizontale Rahmenelemente 26, 27 auf, wobei das Rahmenelement 26 im Betrieb der Bodenplatte des Fermenters zugewandt und länger gebildet ist als das im Betrieb dem oberen Ende des Aufnahmevolumens zugewandte horizontale Rahmenelement 27, das im Vergleich zu dem Rahmenelement 26 von kürzerer Abmessung ist. Diese horizontalen Rahmenelemente sind miteinander über Verbindungsrahmenelemente 28, 29 verbunden, die schräg zu den beiden horizontalen Rahmenelementen 26, 27 und geneigt zur Vertikalen verlaufen.

Nahe der mittigen Symmetrieachse des zu einer Vertikalachse achssymmetrisch gebildeten Gitterrahmenrührers 15 verlaufen zwei Vertikalstützen 30, 31, die den Rahmen 25 stabilisieren, diesen in zwei Rahmenelemente unterteilen und zudem Festlegungspunkte für später noch zu beschreibende weitere Elemente bieten.

In im Wesentlichen äquidistanter Verteilung sind entlang der horizontalen Erstreckung des Rahmens zueinander beabstandet jeweils vertikal verlaufende Längsstreben 32 angeordnet. Diese sind mit jeweils einem Ende an dem oberen horizontalen Rahmenelement 27 und einem anderen Ende an dem unteren Rahmenelement 26 festgelegt. Bei diesen Längsstreben 32 handelt es sich um Flachbandstreben, die - und dies ist eine Besonderheit des Rahmenrührers 15 - einmal um 180° um ihre Längsachse gedreht verlaufen. Hierdurch bewirken diese Längsstreben 32 bei einer Rotation des Gitterrahmenrührers 15 eine verbesserte Durchmendung und Durchmischung des organischen Ausgangsmaterials mit den bioaktiven Kulturen.

Zwischen den beiden Vertikalstützen 30, 31 sind zwei kürzere Querstreben 33 angeordnet, die ebenfalls aus einem Flachbandmaterial gebildet sind, welche entlang seiner Längserstreckung einmal um 180° verdreht ist. Diese Querstreben 33 unterteilen die Höhe des Gitterrahmenrührers in drei etwa gleiche Abschnitte und sind insoweit über diese Höhe im Wesentlichen äquidistant angeordnet.

Diese besondere in Fig. 6 dargestellte Gestaltung des Rührers als Gitterrahmenrührer 15 mit den aus einem Flachbandmaterial gebildeten Längs- und Querstreben, die den trapezförmig aufgebauten Rahmen 25 durchspannen und jeweils um ihre Längsachse um 180° verdreht bzw. verdrillt verlaufen, haben in den Versuchsergebnissen der Erfinder den besonders guten Durchmischungseffekt bei gleichzeitiger ausreichender "Ruhe" für die biologisch aktiven Mikroorganismen gezeigt, so dass letztere sehr effizient die Umsetzung des organischen Ausgangsmaterials zu methanhaltigem Biogas betreiben konnten.

### Bezugszeichenliste

- 1: Fermenter
- 2: Fermenterbehälter
- 3: Aufnahmevolumen
- 4: Bodenplatte
- 5: Seitenwand
- 6: Abdeckung
- 7: Gasreservoir
- 8: äußere Membran
- 9: innere Membran
- 10: Gebläse
- 11: Netzabdeckung
- 12: Antriebsmotor
- 13: Antriebswelle
- 14: Mittelachse
- 15: Gitterrahmenrührer
- 16: Laufschiene
- 17: Revisionsöffnung
- 18: Füllstandsanzeige
- 19: Absaugung
- 20: Isolierschicht
- 21: Schutzbeschichtung mit Antihaft-Effekt
- 22: Isolierung
- 23: Schraubbolzen
- 24: Dichtung
- 25: Rahmen
- 26: horizontales Rahmenelement
- 27: horizontales Rahmenelement
- 28: Verbindungsrahmenelement
- 29: Verbindungsrahmenelement
- 30: Vertikalstütze
- 31: Vertikalstütze
- 32: Längsstrebe
- 33: Querstrebe
- 100: Fermenter
- 106: Abdeckung

## Patentansprüche

1. Fermenter für eine Anlage zur Umwandlung organischen Ausgangsmaterials in methanhaltiges Biogas mit:
- einem eine kreiszylinderförmige umlaufende Seitenwand (5) und einen diese nach unten hin abschließenden Boden (4) aufweisenden, ein Aufnahmevolumen (3) definierenden Fermenterbehältern (2);
- einer Abdeckung (6; 106) zum gasdichten Abschluss des Fermenterbehälters (2) und zum Zurückhalten von entstehendem Biogas;
- einem Rührwerk zum Umwälzen von in dem Aufnahmevolumen (3) befindlichem organischen Ausgangsmaterial und in diesem befindlichen bioaktiven Kulturen;
- einer Speiseleitung zum Zuführen frischen organischen Ausgangsmaterials in das Aufnahmevolumen (3);
- einer Heizung zum Erwärmen und/oder Warmhalten des in dem Aufnahmevolumen (3) befindlichen organischen Ausgangsmaterials;
- einem Ablauf für verbrauchtes organisches Ausgangsmaterial;
- einem Abzug zur Entnahme von Biogas aus dem Fermenterbehälter (2) und
- einer Belüftung zum Zuführen von Luft in das Aufnahmevolumen (3) zum Belüften des darin gehaltenen organischen Ausgangsmaterials,
wobei das Aufnahmevolumen (3) durch den Boden (4) und die zylindrische Seitenwand (5) glattwandig und ohne jegliche Anbauteile an Boden (4) und/oder Seitenwand (5) in diesem Bereich begrenzt ist,
wobei die Heizung eine Fußbodenheizung in dem Boden (4) umfasst,
wobei das Rührwerk durch einen um eine Längsachse (14) der zylinderförmigen Seitenwand (5) rotierbaren Gitterrahmenrührer (15) gebildet ist mit Rührgitterrahmen, die sich ausgehend von der Längsachse (14) über im Wesentlichen die vollständige Füllhöhe des Aufnahmevolumens (3) und, zumindest im Bereich des Bodens (4), über im Wesentlichen den gesamten Radius des durch die Seitenwand (5) seitlich begrenzten Aufnahmevolumens (3) erstrecken,
wobei die Speiseleitung zum Zuführen frischen organischen Ausgangsmaterials in das Aufnahmevolumen (3) wenigstens eine Druck für die Zufuhr des Ausgangsmaterials aufbauende Druckpumpe aufweist und wobei die Speiseleitung in wenigstens einer, vorzugsweise mehreren oberhalb des Aufnahmevolumens (3) und oberhalb des Gitterrahmenrührers (15) angeordneten Einspritzdüsen mündet.

2. Fermenter nach Anspruch 1, **dadurch gekennzeichnet, dass** die das Aufnahmevolumen (3) begrenzenden Oberflächen der Seitenwand (5) und des Bodens (4) für eine Haftungsverminderung behandelt, insbesondere beschichtet, sind.

3. Fermenter nach einem der Ansprüche 1 oder 2, **dadurch gekennzeichnet, dass** die Belüftung eine Luftführung im Boden (4) und eine Vielzahl von in dem Boden (4) gebildete Eingasdüsen umfasst, wobei in dem Boden (4) geführte Luftleitungen so in Kontakt mit in dem Boden (4) eingelassenen Rohren der Fußbodenheizung gebracht sind, dass im Betrieb durch in den Rohren der Fußbodenheizung geführtes Heizmedium die Luft in den Luftleitungen vorerwärmt wird, bevor diese durch die Eingasdüsen in das Aufnahmevolumen (3) einströmt.

4. Fermenter nach einem der vorhergehenden Ansprüche, **dadurch gekennzeichnet, dass** der Gitterrahmenrüher (15) zweiflügelig und symmetrisch zu der Längsachse (14) gebildet ist.

5. Fermenter nach einem der vorhergehenden Ansprüche, **dadurch gekennzeichnet, dass** die Rührgitterahmen des Gitterrahmenrührers (15) je eine äußere Rahmenkonstruktion (26, 27, 28, 29) und dazwischen verlaufende Längs- (32) und Querstreben (33) aufweisen, wobei die Längsstreben (32) im Wesentlichen vertikal, die Querstreben (33) im Wesentlichen horizontal verlaufen.

6. Fermenter nach Anspruch 5, **dadurch gekennzeichnet, dass** die Längsstreben (32) in den jeweiligen Rührgitterrahmen derart angeordnet sind, dass sie diesen in regelmäßigen Abständen entlang eines horizontalen Verlaufes von der Längsachse (14) gesehen nach außen unterteilen.

7. Fermenter nach einem der Ansprüche 5 oder 6, **dadurch gekennzeichnet, dass** die Längs- (32) und Querstreben (33) durch Flachbandmaterial gebildet sind und entlang ihrer jeweiligen Längsachse je wenigstens einmal um 180° verdreht sind.

8. Fermenter nach einem der vorhergehenden Ansprüche, **dadurch gekennzeichnet, dass** ein Antriebsmotor (12) für den Giterrahmenrührer (15) außerhalb des Fermenterbehälters (2) angeordnet und über durch den Fermenterbehälter (2) geführte Antriebswellen (13) mit dem Giterrahmenrüher (15) wirkverbunden ist.

9. Fermenter nach einem der vorhergehenden Ansprüche, **dadurch gekennzeichnet, dass** in der Seitenwand (5) eine Wandheizung angeordnet ist zum Erwärmen bzw. zum Warmhalten des in dem Aufnahmevolumen befindlichen organischen Ausgangsmaterials.

10. Fermenter nach Anspruch 8, **dadurch gekennzeichnet, dass** die Wandheizung mit der Fußbodenheizung zu einem gemeinsamen Heizkreislauf verbunden ist.

11. Fermenter nach einem der vorhergehenden Ansprüche, **gekennzeichnet durch** einen Fermenterbehälter (2) mit einem das Aufnahmevolumen (3) begrenzenden Innendurchmesser von zwischen 2m und 2,5m, insbesondere von 2,2m, und mit einer Füllhöhe des Aufnahmevolumens (3) von zwischen 0,6m und 1,0m, insbesondere von 0,8m.

12. Fermenter nach einem der vorhergehenden Ansprüche, **dadurch gekennzeichnet, dass** die Abdeckung eine flexible Abdeckung (6), insbesondere eine Folienabdeckung, ist.

13. Fermenter nach einem der Ansprüche 1 bis 11, **dadurch gekennzeichnet, dass** die Abdeckung eine starre, insbesondere plattenförmige, Abdeckung (106) ist.

14. Biogasanlage mit wenigstens einem Fermenter (1; 100) nach einem der vorhergehenden Ansprüche.
